Europäisches Patentamt

European Patent Office

Office européen des brevets

(19)

(11) **EP 1 028 107 A1**

(12) **EUROPÄISCHE PATENTANMELDUNG**

(43) Veröffentlichungstag:
**16.08.2000 Patentblatt 2000/33**

(51) Int Cl.⁷: **C07C 303/10**, C07C 309/80,
C07C 303/14, C07C 309/04

(21) Anmeldenummer: **00102039.5**

(22) Anmeldetag: **02.02.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **11.02.1999 DE 19905613**

(71) Anmelder: **Aventis Research & Technologies GmbH & Co. KG**
**65926 Frankfurt am Main (DE)**

(72) Erfinder:
• **Hagemeyer, Alfred, Dr.**
**48431 Rheine (DE)**
• **Kühlein, Klaus, Prof.Dr.**
**65779 Kelkheim (DE)**

(54) **Photoinitiierung der Sulfochlorierung oder Sulfoxidation von Alkanen durch UV-Excimer-Strahlung zur Herstellung von Alkansulfochloriden und Alkansulfonaten**

(57) Verfahren zur Herstellung von Alkansulfochloriden und Alkansulfonaten durch Umsetzung von Alkanen mit $SO_2$ und $Cl_2$ oder $O_2$ zu Alkansulfochloriden oder Alkansulfonaten, wobei die Reaktion durch UV-Excimer-Strahlung photoinitiiert wird. Geeignete Wellenlängen der UV-Excimer-Strahlung liegen im Bereich von 150-470nm.

Vorzugsweise werden folgende Wellenlängen (Excimere) verwendet: 172nm ($Xe_2$*), 193nm (ArF*), 222nm (KrCl*), 248nm (KrF*), 282nm (XeBr*), 291nm ($Br_2$*), 308nm (XeCl*), 342nm ($I_2$*), 351nm (XeF*).

EP 1 028 107 A1

**Beschreibung**

Photoinitiierung der Sulfochlorierung oder Sulfoxidation von Alkanen durch UV-Excimer-Strahlung zur Herstellung von Alkansulfochloriden und Alkansulfonaten

Die Erfindung betrifft die Photoinitiierung der Sulfochlorierung bzw. Sulfoxidation, d.h. der Umsetzung von Alkanen mit $SO_2$ und $Cl_2$ bzw. $O_2$ zu Alkansulfochloriden bzw. Alkansulfonaten, durch UV-Excimer-Strahlung.

Die Photoinitiierung chemischer Reaktionen, die nach einem Radikalkettenmechanismus ablaufen, ist bekannt.

In den bekannten Arbeiten zur Photoinitiierung werden sowohl UV-Lichtquellen als auch Lichtquellen, die sichtbares Licht aussenden, eingesetzt. Da die Absorptionskoeffizienten der im erfindungsgemäßen Verfahren verwendeten Stoffe im ultravioletten Spektralbereich höher sind als im sichtbaren Spektralbereich, kann mit entsprechend niedrigeren Leistungsdichten gearbeitet werden, falls UV-Lichtquellen eingesetzt werden. Da dadurch ein wesentlich höherer Durchsatz erreicht wird, ist der Einsatz von UV-Lichtquellen sinnvoll.

Zur Erzeugung von UV-Strahlung werden Lampen, Laser oder sonstige Strahlungsquellen wie Elektronenspeicherringe (Synchrotrons) oder Plasmaentladungen eingesetzt. Bei der Benutzung von Lampen stehen vor allem Hg-Dampflampen (mit starken Emissionslinien bei einer Wellenlänge von 185 nm und 254 nm) zur Verfügung. Weitere Quellen für UV-Strahlung sind Sychrotrons, die eine breitbandige Strahlung bis in den Röntgenbereich liefern, und das Licht einer Plasmaentladung bei niedrigem Druck.

Die Sulfochlorierung gehört ebenso wie die Sulfoxidation zu den wenigen seit vielen Jahren großtechnisch genutzten photochemischen Verfahren zur Herstellung von Alkansulfonaten. Zur Photoinitiierung werden bei allen Produzenten konventionelle UV-Hg-Lampen eingesetzt. Bei dem von Hoechst und Clariant betriebenen Licht-Wasser-Verfahren kommen Quecksilber-Hochdruck-Tauchlampen aus Quarz zum Einsatz.

Alkansulfonate sind wichtige Tenside und werden daher großtechnisch hergestellt. Dabei kommen zwei (photochemische) Verfahren zur Anwendung, die Sulfochlorierung oder die Sulfoxidation.

Der Stand der Technik ist ausführlich in H.G. Hauthal, "Alkansulfonate", VEB Deutscher Verlag für Grundstoffindustrie, Leipzig, 1985 dargestellt. Die theoretischen Zusammenhänge der Reaktionen wurden inzwischen aufgeklärt und sind in der o.g. Monographie zu finden. Die Reaktionen verlaufen nach einem Radikalkettenmechanismus.

Bei der Sulfochlorierung von n-Alkanen wird das n-Alkan mit $SO_2$ und $Cl_2$ zur Reaktion gebracht. Obgleich zum Kettenstart außer Licht auch chemische Radikalbildner und $\gamma$-Strahlen geeignet sind, wird die Sulfochlorierung technisch bis heute nur unter Lichteinwirkung durchgeführt.

$$RH + SO_2 + Cl_2 \rightarrow RSO_2Cl + HCl$$

Die Sulfochlorierung verläuft nach folgendem Mechanismus:

$$Cl_2 \rightarrow 2\ Cl.$$

$$RH + Cl. \rightarrow R. + HCl$$

$$R. + SO_2 \rightarrow RSO_2.$$

$$RSO_2. + Cl_2 \rightarrow RSO_2Cl + Cl.$$

Die Energie der zur Sulfochlorierung erforderlichen Lichtstrahlung muß mindestens der Bindungsenergie im $Cl_2$ entsprechen. Als weitere Voraussetzung zum Kettenstart (Bildung von Cl-Starterradikalen durch Photodissoziation von $Cl_2$) muß die Strahlung mit ausreichendem Energieinhalt auch vom Chlor absorbiert werden. UV-Strahlen mit einer Wellenlänge unterhalb ca. 320nm sind nicht geeignet, da Chlor in diesem Bereich nicht absorbiert bzw. völlig durch Schwefeldioxid beschattet wird. Am besten arbeitet man mit kurzwelligem sichtbarem Licht bzw. langwelligem UV zwischen 370 und 478nm. Die Grenzwellenlänge, oberhalb derer eine Photodissoziation der $Cl_2$-Bindung energetisch nicht mehr möglich ist, beträgt 478,8nm.

Schumacher, Stauff, Chemie 55 (1942) 341; Z. Elektrochem. 48 (1942) 550, verwendeten für ihre Versuche Licht der Wellenlänge 436nm. Die Arbeiten von Asinger et al., Ber. Dtsch. Chem. Ges. 75 (1942) 34; Ber. Dtsch. Chem. Ges. 75 (1942) 42; Ber. Dtsch. Chem. Ges. 75 (1942) 344; Ber. Dtsch. Chem. Ges. 77 (1944) 191; J. prakt. Chem. (4) 2 (1955) 37; J. prakt. Chem. (4) 2 (1955) 203; J. prakt. Chem. (4) 2 (1955) 228; J. prakt. Chem. (4) 2 (1955) 233; Chem. Ber. 91 (1958) 2130; Chem. Ber. 96 (1963) 2831, vermitteln demgegenüber den Eindruck, als wäre es notwendig, bei der Sulfochlorierung UV-Licht zu verwenden.

Die Reaktionstemperatur, bei der die Sulfochlorierung betrieben wird, kann in Abhängigkeit vom Einsatzprodukt (Alkan) in gewissen Grenzen variiert werden. Sie sollte jedoch 35 bis 40°C nicht überschreiten, da sich dann in steigendem Maße chlorierte Nebenprodukte bilden. Die erzielbaren Selektivitäten hängen u.a. vom Molverhältnis $SO_2/Cl_2$ ab.

Alle bei Raumtemperatur gasförmigen, flüssigen und festen n-Alkane lassen sich glatt sulfochlorieren, ebenso alle Cycloalkane. Isoalkane sind gleichfalls geeignet, ergeben aber höhere Anteile an chlorierten Produkten.

Gasförmige Alkane können entweder bis herab zu -80°C in kondensierter Phase oder in CCl$_4$ gelöst sulfochloriert werden. Hochschmelzende Alkane löst man ebenfalls in CCl$_4$. Auch Sulfochlorierung in Suspension ist möglich.

Die durch Sulfochlorierung primär erhältlichen Alkansulfochloride können nach bekannten Verfahren (z.B. Verseifung mit Lauge nach R-SO$_2$Cl + 2 NaOH → R-SO$_3$Na + NaCl + H$_2$O) zu den gewünschten Alkansulfonaten weiterverarbeitet werden. Sulfochloride können auch zu Weichmachern vom Typ Alkansulfonsäurearylester weiterverarbeitet werden.

Zur technischen Durchführung der Sulfochlorierung werden typischerweise Tauchlampen eingesetzt. Dabei werden einseitig geschlossene Rohre aus starkwandigem Glas ein den Reaktionskessel eingeführt, die zur Aufnahme der breitbandigen, vorwiegend im kurzwelligen sichtbaren Bereich bzw. langwelligem UV-Bereich strahlenden Spezialleuchtröhren dienen.

Bei der Sulfoxidation von n-Alkanen entstehen durch Umsetzung des n-Alkans mit einem Gemisch von Schwefeldioxid und Sauerstoff unter Einstrahlung von ultraviolettem Licht Alkansulfonsäuren. Die Reaktion ist exotherm und verläuft nach der Bruttogleichung

$$2\ RH + 2\ SO_2 + O_2 \rightarrow 2\ RSO_3H$$

Durch Neutralisation der Sulfonsäuren nach der Reaktionsgleichung

$$RSO_3H + NaOH \rightarrow RSO_3Na + H_2O$$

werden Produkte erhalten, die den Sulfonaten auf Basis Sulfochlorierung ähnlich sind. Die Sulfoxidation stellt deshalb eine ohne den teuren Rohstoff Chlor arbeitende Alternative zur Sulfochlorierung dar und benötigt zudem nicht 2 Mole Natriumhydroxid pro Mol Alkansulfonat, sondern nur 1 Mol. Sehr vorteilhaft ist auch die vollständige Umwandlung der eingesetzte Rohstoffe zu Sulfonsäuren ohne stöchiometrisch bedingte Bildung von Nebenprodukten.

Allerdings läuft die Sulfoxidation nicht so einfach ab, wie es nach der Bruttogleichung den Anschein haben könnte. Durch Folgereaktionen bildet sich schon bei niedrigen Umsätzen sehr leicht eine schwere Sulfonsäurephase, die einen erheblichen Anteil an Di- und Polysulfonsäuren sowie Schwefelsäure enthält. In dieser elementaren Form ist die Sulfoxidation daher technisch nicht verwertbar.

Von zahlreichen untersuchten Verfahrensvarianten wurden das Essigsäureanhydrid-Verfahren und das Licht-Wasser-Verfahren so weit entwickelt, daß kleintechnische Anlagen errichtet werden konnten. Den entscheidenden Durchbruch erzielte die Hoechst AG ab Mitte der sechziger Jahre mit der Weiterentwicklung des Licht-Wasser-Verfahrens zur großtechnischen Reife.

Die Jahreskapazität beträgt mehr als 100.000 t Alkansulfonat nach diesem Verfahren.

Die Sulfoxidation verläuft nach einem Radikalkettenmechanismus, dessen Schritte inzwischen aufgeklärt wurden. Zum Kettenstart werden Alkylradikale R. benötigt. Paraffine und auch Sauerstoff zeigen im angewandten Spektralbereich der UV-Quarzlampen keine Absorption. Der primäre Schritt besteht in der Einwirkung des UV-Lichts auf Schwefeldioxid. Die Absorption von UV-Strahlung durch Schwefeldioxid und die Reaktion des angeregten SO$_2$ mit dem Alkan wurde von J.G. Calvert et al., J. Amer. Chem. Soc. 93 (1971) 3115, untersucht. Der entscheidende Prozeß besteht in der Anregung des SO$_2$ durch UV-Licht >320nm zum Triplett-SO$_2$ ($^3$SO$_2$). Dieses reagiert mit Alkanen quantitativ nach

$$^3SO_2 + RH \rightarrow R. + HSO_2.$$

Kürzere Wellenlängen (240 bis 320nm) regen das SO$_2$ zum Singulett-SO$_2$ ($^1$SO$_2$) an, welches nicht mit Kohlenwasserstoffen reagiert. Nur nach dem Übergang des $^1$SO$_2$ zum $^3$SO$_2$, welcher zu etwa 9% erfolgt, ist eine Reaktion mit dem Alkan möglich. Dann läuft folgende Reaktionskette ab:

$$R. + SO_2 \rightarrow RSO_2.$$

$$RSO_2. + O_2 \rightarrow RSO_2OO.$$

$$RSO_2OO. + RH \rightarrow RSO_2OOH + R.$$

Aus einem Alkylradikal entstehen ein Molekül Alkanpersulfonsäure und ein neues Alkylradikal, welches die Reaktionskette erneut auslöst. Die Alkanpersulfonsäure ist instabil und Ausgangspunkt für weitere Schritte:

$$RSO_2OOH \rightarrow RSO_2O. + HO.$$

$$RSO_2O. + RH \rightarrow RSO_2OH + R.$$

$$HO. + RH \text{ -> } H_2O + R.$$

Ein Molekül Persäure liefert also im weiteren Reaktionsverlauf neben je einem Molekül Alkansulfonsäure und Wasser 2 Alkylradikale, so daß man sehr hohe Quantenausbeuten erzielt.

Ein Teil der Alkanpersulfonsäure reagiert mit Schwefeldioxid und Wasser nach folgender Reaktionsgleichung:

$$RSO_2OOH + SO_2 + H_2O \rightarrow RSO_2OH + H_2SO_4$$

Beim Licht-Wasser-Verfahren wendet man die Reduktion der Sulfopersäure durch $SO_2$ und Wasser absichtlich an.

Die Initiierung der Sulfoxidation kann durch chemische Radikalbildner oder mit Hilfe physikalischer Vorgänge erfolgen. Besonders günstig ist die Initiierung durch UV-Licht. Dazu verwendet man Hoch- oder Mitteldruck-Quecksilberlampen aus Quarz, deren Leistung bis zu 60 kW betragen kann.

Beim Licht-Wasser-Verfahren der Hoechst AG ist der Reaktor mit Quecksilber-Hochdruck-Tauchlampen aus Quarz bestückt. Unter der über den gesamten Reaktorquerschnitt wirkenden intensiven UV-Strahlung erfolgt bei 30 bis 38°C die Sulfoxidation, wobei nur ein geringer Teil des Paraffins reagiert und auch das Kreislaufgas aus $SO_2$ und $O_2$ nur zum Teil umgesetzt wird. Die primär gebildete Alkanpersulfonsäure geht durch Reaktion mit Schwefeldioxid und Wasser sofort in Alkansulfonsäure und Schwefelsäure über. Da die Persäure nur intermediär auftritt, kann sie nicht unkontrolliert zerfallen.

Technisch werden also bei der Sulfochlorierung und Sulfoxidation zur Photoinitiierung ausschließlich Hg-Dampflampen als UV-Strahlungsquellen eingesetzt.
Hg-Dampflampen sind breitbandige Strahlungsquellen mit Spektralanteilen sowohl im kurzwelligen VUV-Bereich als auch im sichtbaren Bereich. Die Leistungsdichte im erforderlichen (für die Photoinitiierung wirksamen) Spektralbereich von 320 - 470 nm ist also wesentlich geringer als die Nennleistung der Lampen. Ferner können die unerwünschten kurz- und langwelligen Spektralanteile zu Nebenreaktionen, Ablagerungen auf den Strahlungsquellen bzw. Reaktorwandungen oder Erwärmungen führen und müssen daher ausgefiltert werden (Energievernichtung durch aufwendige Filter- und Kühlperipherie).

Die Aufgabe der vorliegenden Erfindung ist es daher ein Verfahren zur Verfügung zu stellen, das die obengenannten Nachteile vermeidet.

Die vorliegende Erfindung betrifft somit ein Verfahren zur Herstellung von Alkansulfochloriden und Alkansulfonaten durch Umsetzung von Alkanen mit $SO_2$ und $Cl_2$ oder $O_2$ zu Alkansulfochloriden oder Alkansulfonaten und Photoinitiierung der Reaktion UV-Excimer-Strahlung.

Im Vergleich zu den konventionellen breitbandigen Hg-Lampen haben monochromatische UV-Excimer-Strahlungsquellen wie gepulste Excimer-Laser und inkohärente Excimer-Lampen den Vorteil, daß gezielt auf der "richtigen" (d.h. für die Photoinitiierung wirksamsten) Wellenlänge eingestrahlt werden kann und somit die unerwünschten kurz- und langwelligen Spektralanteile der Hg-Lampen vermieden werden.

Die erfindungsgemäß verwendbaren Eximerlampen zeichnen sich durch eine enge spektrale Begrenzung aus Für die Erfindung kommt UV-Excimerstrahlung zum Einsatz, die vorteilhafterweise monochromatisch ist und hohe Leistungsspitzen aufweist. Geeignete Wellenlängen liegen im Bereich von 150 bis 470 nm.

Bevorzugte Wellenlängen sind 172 nm ($Xe_2$*), 193 nm (ArF*), 222 nm (KrCl*), 248 nm (KrF*), 282 nm (XeBr*), 291 nm ($Br_2$*), 308 nm (XeCl*), 342 nm ($I_2$*) und 351 nm (XeF*).
Geeignete UV-Intensitäten liegen zwischen 0,01 und 100 W/cm². Bevorzugte Parameter sind UV-Strahlungsleistungen zwischen 0,05 und 20 W/cm². Bei Einsatz gepulster Laser liegen die geeigneten Pulsfrequenzen im Bereich zwischen 0,1 und 5000 Pulse/s.

Die UV-Strahlung wird durch den Zerfall von Excimeren oder Exciplexen wie $Kr_2$* (Wellenlänge 146 nm), $Xe_2$* (172 nm), KrCl* (222 nm) oder XeCl* (308 nm) erzeugt. Als leistungsstarke UV-Laser werden gepulste Excimerlaser eingesetzt. Auch hier entsteht das Licht durch den Zerfall von Excimeren oder Exciplexen wie $F_2$* (154 nm), ArF* (193 nm), KrF* (248 nm), XeCl* (308 nm) und XeF* (351 nm). Es können auch frequenzvervielfachte Nd-YAG:Laser (Wellenlänge 1064 nm / n; (n = 3, 4, 5,6, 7, 8, 9) verwendet werden.

Die vorliegende Erfindung basiert auf monochromatischer UV-Excimer-Strahlung zur Photoinitiierung. Das erfindungsgemäße Verfahren arbeitet sehr selektiv, ist leicht steuerbar und erfordert keine aufwendigen Apparaturen, da sowohl Excimer-Laser als auch Excimer-Lampen-Module als Komplettsysteme kommerziell erhältlich sind. Da Excimerstrahlung aufgrund der Physik ihrer Erzeugung immer monochromatisch ist und die optimale (diskrete) Wellenlänge vom Experimentator wählbar ist, entfallen die für Hg-Lampen notwendige komplizierte Filter- und Kühl-Peripherie, so daß Excimerstrahler weniger aufwendig, wesentlich kompakter, sparsamer im Energieverbrauch, leicht steuerbar und einfach zu bedienen sind.

Erfindungsgemäße UV-Strahlungsquellen sind Excimer-Strahlungsquellen wie gepulste Excimer-Laser oder inkohärente Excimer-Lampen. Besonders bevorzugt sind Excimer-Lampen. Gegenüber den Excimer-Lasern zeichnen sich Excimer-Lampen durch größere Zuverlässigkeit, einfachere Handhabung und geringere Betriebskosten aus.

Bei monochromatischen UV-Excimer-Strahlern stehen im Spektralbereich zwischen 250 und 450 nm etwa 20 diskrete Wellenlängen zur Verfügung. Dabei können Leistungen bis zu einigen kW erreicht werden.

Das Prinzip der Excimer-Lampe wurde vor mehr als 100 Jahren von Werner von Siemens erfunden. Eine Vielzahl von Methoden zur Erzeugung inkohärenter Excimerstrahlung ist untersucht worden, wobei ein spezieller Entladungstyp für industrielle Anwendungen großes Potential besitzt (Kogelschatz, Esrom, Laser und Optoelektronik 22(4) (1990) 55-59). Bei diesem neuen inkohärenten UV-Strahler werden Excimere in einer stillen elektrischen Entladung gebildet, einer Nichtgleichgewichtsentladung, die gelegentlich auch als Barrierenentladung bezeichnet wird. Das wichtigste Merkmal dieses Entladungstyps ist die Existenz (mindestens) einer dielektrischen Barriere, also eines Isolators, im Strompfad zwischen den Elektroden. Das Dielektrikum (z.B. Quarzrohr) bewirkt eine kapazitive Kopplung zwischen der treibenden elektrischen Wechselspannung und dem Entladungsplasma. Eine robuste Bauweise wird z.B. durch zwei koaxial angeordnete Quarzrohre ermöglicht, wobei ein ringförmiger Entladungsspalt entsteht. Die Quarzwände dienen gleichzeitig als Dielektrika. Beide Elektroden befinden sich außerhalb des Entaldungsraumes und kommen mit dem Plasma nicht in Berührung. Die UV-Strahlung kann nach innen oder nach außen (durch die z.B. als Drahtnetz ausgebildeten Elektroden) austreten. Die erreichbaren Leistungsdichten liegen mittlerweile (neuester Stand bei Heraeus, 1998) bei 200 mW/cm$^2$ bei einer Bandbreite von nur ca. 5 nm. Durch Parallelschaltung mehrerer Entladungsröhren lassen sich sehr hohe UV-Strahlungsleistungen erzielen.

Die im Vergleich zum Laser fehlende Kohärenz der Strahlung spielt für die Materialbearbeitung und für photochemische Anwendungen keine Rolle. Auch das zeitliche Verhalten unterscheidet sich wesentlich von anderen konventionellen UV-Quellen. Der Excimerstrahler sendet Pakete von äußerst kurzen intensiven UV-Strahlungspulsen aus von jeweils nur einigen Nanosekunden Pulsdauer. Durch die äußere Versorgungsquelle kann sowohl die Intensität als auch die Dauer der Emissionsperioden gesteuert werden. Ein weiteres wichtiges Merkmal der Entladungsröhre ist der geschlossene Entladungsraum, der ausschließlich durch Quarzwände begrenzt wird. Dadurch ist die für die Excimerbildung erforderliche Reinheit der Gase über längere Zeit gewährleistet.

Zur Durchführung der Reaktion kann man beispielsweise einen Reaktor mit Tauchlampe(n) verwenden, der aus einem Behälter für die Reaktionsteilnehmer besteht, in den eine oder mehrere UV-Excimerlampen eintauchen, die sich in einem oder mehreren Lampenträgern aus Quarz befinden. Man kann für diese Reaktion auch einen optischen Aufbau verwenden, der mehrere Lampen enthält, die außen um den Behälter für die Reaktionsteilnehmer (z.B. zylindrisches Rohr aus UV-durchlässigem Quarz) angeordnet sind.

Die Reaktion kann sowohl kontinuierlich wie diskontinuierlich durchgeführt werden. Die Isolierung der entstandenen Produkte kann nach bekannten Methoden erfolgen.

So kann man beispielsweise auch gleichzeitig mehrere Wellenlängen (mit mehreren Excimer-Strahlern) einstrahlen. Ferner können mehrere Lampen parallel und in Serie zusammengeschaltet werden. Die Photoinitiierung mit Excimer-Strahlung kann örtlich durchgehend oder abschnittsweise und zeitlich kontinuierlich oder intermittierend erfolgen. Denkbar ist auch der gleichzeitige Einsatz von herkömmlichen Hg-Lampen und erfindungsgemäßen Excimer-Lampen, also eine teilweise Umrüstung bzw. Aufrüstung der Anlagen mit zusätzlichen Excimerlampen.

**Patentansprüche**

1. Verfahren zur Herstellung von Alkansulfochloriden und Alkansulfonaten durch Umsetzung von Alkanen mit $SO_2$ und $Cl_2$ oder $O_2$ zu Alkansulfochloriden oder Alkansulfonaten, dadurch gekennzeichnet, daß die Reaktion durch UV-Excimer-Strahlung photoinitiiert wird.

2. Verfahren nach Anspruch 1, dadurch gekennzeichnet, daß die UV-Excimer-Strahlung mittels gepulster Excimer-Laser erzeugt wird

3. Verfahren nach Anspruch 1, dadurch gekennzeichnet, daß die UV-Excimer-Strahlung mittels inkohärenter Excimer-Lampen erzeugt wird.

4. Verfahren nach einem der vorhergehenden Ansprüche, dadurch gekennzeichnet, daß die Wellenlängen der UV-Excimer-Strahlung im Bereich von 150 - 470 nm liegen.

5. Verfahren nach einem der vorhergehenden Ansprüche, dadurch gekennzeichnet, daß eine der folgenden Wellenlängen (Excimere) verwendet wird: 172 nm ($Xe_2$*), 193 nm (ArF*), 222 nm (KrCl*), 248 nm (KrF*), 282 nm (XeBr*), 291 nm ($Br_2$*), 308 nm (XeCl*), 342 nm ($I_2$*), 351 nm (XeF*).

**Europäisches Patentamt**

## EUROPÄISCHER RECHERCHENBERICHT

Nummer der Anmeldung

EP 00 10 2039

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | EP 0 424 762 A (BAYER AG) 2. Mai 1991 (1991-05-02) * Zusammenfassung; Anspruch 1; Beispiel 1 * --- | 1-5 | C07C303/10 C07C309/80 C07C303/14 C07C309/04 |
| A | DATABASE WPI Section Ch, Week 199237 Derwent Publications Ltd., London, GB; Class D25, AN 1992-306698 XP002139135 & SU 1 685 929 A (VOLG KHIMPROM PRODN ASSOC), 23. Oktober 1991 (1991-10-23) * Zusammenfassung * --- | 1-5 | |
| A | DATABASE WPI Section Ch, Week 199033 Derwent Publications Ltd., London, GB; Class A89, AN 1990-250059 XP002139136 & JP 02 173752 A (JAPAN SYNTHETIC RUBBER CO LTD), 5. Juli 1990 (1990-07-05) * Zusammenfassung * ----- | 1-5 | |

**RECHERCHIERTE SACHGEBIETE (Int.Cl.7)**

C07C

Der vorliegende Recherchenbericht wurde für alle Patentansprüche erstellt

| Recherchenort | Abschlußdatum der Recherche | Prüfer |
|---|---|---|
| MÜNCHEN | 6. Juni 2000 | Arias-Sanz, J |

**ANHANG ZUM EUROPÄISCHEN RECHERCHENBERICHT
ÜBER DIE EUROPÄISCHE PATENTANMELDUNG NR.**

EP 00 10 2039

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-06-2000

| Im Recherchenbericht angeführtes Patentdokument | | Datum der Veröffentlichung | Mitglied(er) der Patentfamilie | | Datum der Veröffentlichung |
|---|---|---|---|---|---|
| EP 0424762 | A | 02-05-1991 | DE | 3935642 A | 02-05-1991 |
| | | | DE | 59003787 D | 20-01-1994 |
| | | | JP | 3145454 A | 20-06-1991 |
| | | | US | 5096625 A | 17-03-1992 |
| SU 1685929 | A | 23-10-1991 | KEINE | | |
| JP 2173752 | A | 05-07-1990 | KEINE | | |

EPO FORM P0461